# EUROPEAN PATENT SPECIFICATION

(11) **EP 2 272 945 B1**
(45) Date of publication and mention of the grant of the patent: **10.09.2014**
(21) Application number: 09008818.8
(22) Date of filing: 06.07.2009
(51) Int. Cl.: C12M 1/42, C12M 3/00, C12N 13/00

(54) **Method and system for the manipulation of cells**
Verfahren und System zur Manipulation von Zellen
Procédé et système pour la manipulation de cellules

(43) Date of publication of application: 12.01.2011
(73) Proprietor: Gottfried Wilhelm Leibniz Universität Hannover, 30167 Hannover (DE); President and Fellows of Harvard College, Cambridge, MA 02138 (US)
(72) Inventor: Mazur, Eric, Prof. Dr., Concord, MA 01742 (US); Heisterkamp, Alexander, Prof. Dr., 30916 Isernhagen (DE); Diebold, Eric, Dr., Cambridge, MA 02139 (US)
(74) Representative: Grünecker, Kinkeldey, Stockmair & Schwanhäusser

(56) References cited:
- EP-A- 1 988 153
- WO-A-2009/017695
- US-A1- 2002 110 847
- US-A1- 2006 079 062

## Description

The present invention is directed to a method according to the preamble of claim 1 for the manipulation of at least one cell, as well as to a corresponding system for the manipulation of at least one cell.

In this context, a "cell" is generally referring to a biological cell, but it could also refer to other biological components than cells, or even to non-biologic, micro-sized objects. Further, a *"manipulation"* refers to a transient or permanent transformation of the cell from one state to another. For example, a cell or components thereof could be excited to a different energy level for diagnostic purposes. In particular, however, the term *"manipulation"* covers techniques such as cell surgery, cell perforation, or cell transfection by the introduction of macromolecules (such as DNA or RNA) into a cell.

A conventional method and apparatus for cell permeabilization are known from US 2005/0095578 A1. According to this document, the membrane of a biological cell may transiently be permeabilized by tightly focusing the pulse of a nanosecond laser onto the cell membrane. Through the resulting hole in the cell membrane, macromolecules such as DNA or RNA strands from the ambience may be introduced into the cell, for example in order to increase or suppress the production of the green fluorescent protein (GFP) in the cell. This conventional technique is also known as *"optoinjection".*

This optoinjection technique has several disadvantages. One disadvantage is that the technique is very slow, as it allows the manipulation of a single cell at any given time only. When a large number of cells are to be manipulated, the environmental conditions may change significantly from the first manipulations to the later ones, thereby rendering the results of the technique unpredictable. Another disadvantage is the high mortality of the cells treated by the optoinjection technique, due to the high stress exerted on the cells.

A different approach is suggested in WO 2009/017695 A1, which discloses a method according to the preamble of claim 1, as well as a corresponding tool for the manipulation of cells. This tool could be a microcapillary or a substrate bearing a plurality of metal nanoparticles. The nanoparticles could either be deposited on the tool directly in the form of particles, or in the form of a film which is subsequently annealed in order to form particles. When the tool is subsequently irradiated with an intense nanosecond laser pulse, the nanoparticles absorb the laser radiation and rapidly heat up by several hundred degrees. This superheating leads to the generation of cavitation bubbles which may locally destroy the membrane of a cell in the vicinity of the nanoparticles.

The method and system of WO 2009/017695 A1 still have disadvantages. For example, the formation of cavitation bubbles is a random process which is not entirely predictable. Depending on their size, the cavitation bubbles will put the biological objects in their vicinity under considerable mechanical stress, which may often lead to cell death. Further, the production of the manipulation tools of WO 2009/017695 A1 is rather complicated and expensive, in particular as it requires an effort to create the nanoparticles.

It is the object of the present invention to further improve the conventional method and system for the manipulation of cells, such that the manipulation system is easy to manufacture, and such that the manipulation yields more reproducible results.

This object is solved by a method with the features of claim 1, by a system with the features of claim 7, and by a use of such a method or such a system according to claim 15.

Advantageous embodiments of the invention are referred to in the dependent claims.

According to the inventive method, surface structures with a size of one micrometer of less are formed on the surface of the substrate prior to depositing the metal on the substrate surface. The metal deposition may be performed by any suitable method, such as evaporation, electrodeposition, sputtering, chemical vapor deposition (CVD), plasma-assisted vapor deposition, or the like. As described in US 2009/0046283 A1, the deposition of a metal onto a surface with a nano-roughness, i.e. with structures of a sub-micrometer size, leads to an inhomogeneous formation of nanoislands or nanoclusters of the metal on the peaks of the rough surface. These nanoislands or nanoclusters of the metal represent the underlying surface roughness, and they have dimensions which are also in the sub-micro meter range. Depending on the shape of the underlying surface structures, the nanoislands or nanoclusters of metal may adopt the shapes of nanorods, nanocones, nanoballs, or irregular shapes. Nothing in US 2009/0046283 A1 suggests that such metalized nanostructures could be used in the manipulation of cells. Compared to the techniques for the formation of nanoparticles disclosed in WO 2009/017695 A1, on the other hand, the method of the present invention offers considerable advantages. For example, the expensive generation and deposition of individual nanoparticles or the energy consuming annealing process can both be avoided. Moreover, the dependency of the formation of nanoislands or nanoclusters on the underlying surface structures offers the advantage of easily controlling and adjusting the shape and dimension of these metal nanoclusters, simply by appropriately controlling the shape and dimensions of the underlying surface structures of the substrate.

When irradiating the surface of the substrate with at least one laser pulse, the membrane of a cell at or near the surface of the substrate can be locally perforated or permeabilized, thereby allowing the introduction of macromolecules or other substances from the ambiance of the cell into its interior. In this context, *"at or near the surface of the substrate"* expresses that the cell is either in contact with the substrate, or at a close enough distance to experience a modulation of the laser radiation by the metal nanostructures on the substrate. For example, this distance could be one or two micrometers, or less than a micrometer.

The cells manipulated by the method according to the invention exhibit a surprisingly high survival rate, as well as a surprisingly high responsiveness to the manipulation. Although specific aspects of the interaction still have to be investigated, this is probably due to using a different mechanism of interaction, as compared to WO 2009/017695 A1. As described above, the technique of WO 2009/017695 A1 relies on the rapid heating of nanoparticles by several hundred degrees, and by the resulting generation of cavitation bubbles, in order to penetrate cell membranes. With the method of the present invention, on the other hand, no or hardly any cavitation bubbles are observed. In contrast to WO 2009/017695 A1, the method of the present invention does not rely on photothermal effects, but on bulk or surface plasmon resonance of the metallic nanoislands or nanoclusters instantaneously leading to an extreme enhancement of the electromagnetic field of the incoming laser radiation. This field is enhanced to such an extent that a sub-micrometer hole is created in the cell membrane of a nearby cell. Due to the spatial confinement of the field enhancement to the immediate vicinity of the nanostructure, components of the cell other than the cell membrane are not affected, thereby reducing cell mortality. Further, the field enhancement occurs within the duration of the laser pulse, and thus, is considerably faster than the photothermal effects used in WO 2009/017695 A1. In other words, the method of the present invention achieves a very high spatial and temporal confinement of the laser cell interaction. As described in detail in US 2009/0014842 A1, or in US 2009/0046283 A1, the surface structures can be formed by laser ablation from the surface of the substrate. The size and shape of the surface structures may be controlled by adjusting the laser parameters (wavelength, pulse duration, number of pulses per spot, energy density, the angle of incidence of the laser radiation on the substrate), or by the ambience of the substrate during laser ablation. For example, different structures can be obtained by performing the ablation in a specific gas atmosphere or in a fluid, for example water. Compared to the prior art, another advantage of the invention is that the nanoislands, as they are not superheated, do not tend to fragmentation. In prior art techniques, on the other hand, metal fragments, smaller than 10nm in size, can intercalate into the DNA and thus lead to mutation or DNA damage.

The method of the present invention also allows to form the surface structures by taking an imprint from a previously formed die. For example, the surface of a silicon blank could be structured by laser ablation, before taking an imprint of the structured surface with a polymer. From this polymer *"negative"* of the structured surface, several substrates could now be formed by means of an injection molding technique. This offers not only the advantage of being able to form several substrates simultaneously, but also of forming them with identical surface structures.

Preferably, the main material of the substrate used in the present invention comprises a plastic material or a semi-conductor material. It is advantageous if this material does not negatively affect biological material in its vicinity, i.e. is biocompatible. In some instances, it may be advantageous if the main material of the substrate (i.e. without the metal coating) is transparent or at least semi-transparent for the laser radiation used for manipulating the cells.

In a preferred embodiment, a plurality of cells is immobilized on the substrate. For example, the cells could be grown on the substrate. It is also conceivable to "suspend" the cells in close proximity to the substrate by inserting appropriate, in particular ferromagnetic particles between the (coated) substrate and the cells. Such particles are offered e.g. by Miltenyi Biotech for stem cell purification. Alternatively, the cells could be suspended above the substrate by a suitable "biological" attachment, for example via antibodies or aptamers.

The method of the present invention becomes particularly efficient if a plurality of surface structures are irradiated by each laser pulse. In contrast to the conventional optoinjection technique, the laser pulses do not have to be tightly focused. Rather, a light focusing or even no focusing at all may be sufficient in order to generate sufficiently high local fields. Hence, a comparatively large area of the substrate may be irradiated by each laser pulse, thereby allowing the simultaneous manipulation of a large number of cells.

Picosecond laser pulses (i.e. pulses with a duration of less than one nanosecond) or femtosecond pulses (i.e. pulses with a duration of less than one picosecond) have turned out to be most usable for the inventive method. Due to the short pulse duration, possibly detrimental photothermal effects as in the conventional techniques can be avoided. Further, the ultrashort laser pulses exhibit higher electromagnetic fields than longer pulses, thereby making the inventive method even more effective.

In the inventive method, consecutive laser pulses can be guided across the surface of the substrate in a predetermined or in a random pattern, in order to subsequently irradiate further areas of the substrate. In order to serve as a reference, certain portions of the substrate may be excluded from irradiation, either by arranging a shadow mask over these portions, or by controlling the scanning pattern accordingly.

The present invention is also directed to system for the manipulation of at least one cell. The system comprises a substrate with a nano-structured surface, onto which a metal is deposited, and a laser for irradiating the surface structures of the substrate. The system may be used for manipulating, in particular permanently or transiently perforating at least one cell which is located on the substrate, or in its close vicinity. As described with respect to the inventive method, the system is easy to manufacture, and offers the advantages of a high responsiveness of the cells, as well as a high survival rate of the cells.

The specific advantages when using a picosecond pulse laser or a femtosecond pulse laser have already been described above. The main body of the substrate (i.e. excluding the metal coating) may comprise a plastic material or a semi-conductor material. The plastic material may be advantageous if the substrate is formed by the above described imprint method.

Preferably, the substrate material is transparent for the laser radiation used for manipulating the cells. This allows to illuminate the nanostructures from the bottom of the substrate, i.e. from the side opposite the surface contacted by the cells, in order to avoid a beam distortion by the cells. In this way, the local field strength is more predictable. Further, a reduced absorption of the laser radiation by the substrate significantly reduces or avoids undesired photothermal effects on the cells due to heating up the substrate.

In an advantageous embodiment, more than 75 per cent of the surface structures on a given area of the substrate have a size of less than 700 nanometers, preferably less than 500 nanometers. Nanoislands or nanoclusters with a size between 150 to 250 nanometers have turned out to be particularly efficient for the cell manipulation with the inventive method or system.

Generally, any type of metal, metallic compound or alloy can be deposited on the substrate surface. Gold, silver, copper, or aluminum are preferred, however, as these metals exhibit strong surface plasmons and a correspondingly high field enhancement effect.

The system of the present invention may also comprise an optical assembly for defining the spot size and the position of the laser radiation on the substrate surface. If desired, this optical assembly may comprise a focusing subassembly, such as a lens, in order to increase the local field strength. The optical assembly may also comprise a scanning system or other deflecting means for directing the laser radiation on specific positions of the substrate surface.

Finally, the invention is also directed by a use of a method as described above, or to a system as described above for the manipulation of at least one cell. In particular, the manipulation may comprise a transfection of the at least one cell, or of a group of cells.

In the following, an advantageous embodiment of the invention will be described with reference to the attached drawings, in which
- Fig. 1: is a schematical vertical section through a substrate,
- Fig. 2: is a schematical section through the substrate after depositing metal thereon,
- Fig. 3: is a section through the coated substrate after arranging cells thereon,
- Fig. 4: is a representation of an embodiment of the system of the present invention during the irradiation with a laser pulse,
- Fig. 5: is a representation of the system after the application of a laser pulse, and
- Fig. 6: is a schematical perspective view of a system according to the invention.

The same components will be referred to throughout all drawings with the same reference numerals.

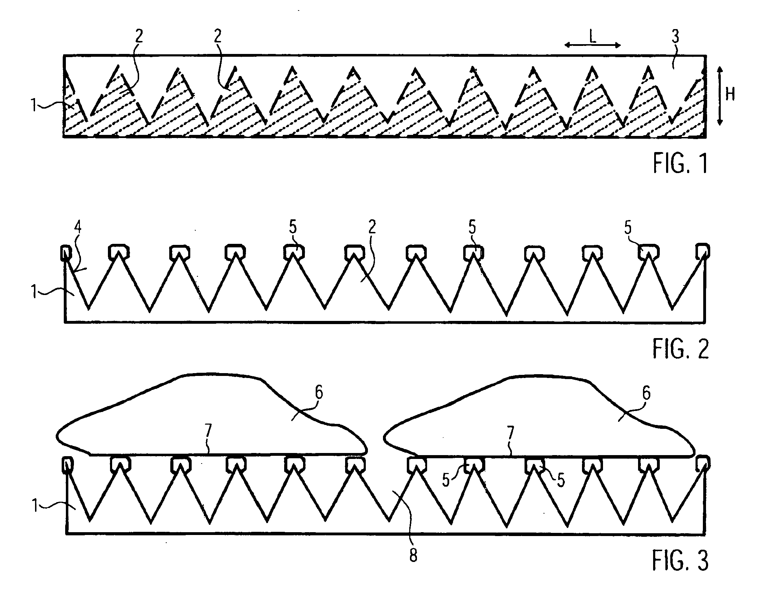

Figure 1 shows a vertical section through a portion of a substrate 1. The substrate 1 may be made from a semiconductor material, for example silicon.

In a first step of the method according to the invention, surface structures 2 are formed on the substrate 1. These surface structures 2 have a size of one micrometer or less, for example 200 to 500 nanometers. In this context, "size" may refer to an amplitude or height dimension H of the surface structures 2, and/or to a lateral dimension L, for example a peak-to-peak distance of the surface structures 2. In Figure 1, these surface structures 2 are schematically shown as individual cone-like peaks on the substrate 1, c.f. the shaded area. However, the shape of the surface structures 2 could be different, in particular more irregular.

A preferred method for creating the surface structures comprises the steps of ablating the surface of the substrate 1 with a short pulse or ultra short pulse laser, for example a Ti:Sapphire laser. The shape of the surface structures 2 depends on the laser parameters (eg. wavelength and pulse duration) as well as the energy density on the substrate 1. The shape and size of the surface structures 2 may also be controlled and modified by performing the laser ablation under a certain gas atmosphere, or with the surface of the substrate 1 placed in a fluid, e.g. water. The area 3 above the surface structures 2 (shown without shading in Figure 1) corresponds to the material removed from the blank substrate 1 by ablation.

The structured substrate 1 could be taken directly for further processing. Alternatively, an imprint method may be used for generating a plurality of similarly structured substrates. For this purpose a negative print of the structured substrate 1 will be taken. For example, a suitable polymer could be used to fill the spaces between and above the surface structures 2 of the substrate 1, which are shown shaded in Figure 1. The polymer could fill the area 3 which was previously removed from the substrate 1 by the ablation process. After suitably curing the polymer, the polymer could serve as a die for generating a plurality of similarly shaped substrates 1, for example by injection molding.

After obtaining the substrate 1 with surface structures 2 having a size of one micrometer or less, a metal is deposited on the surface 4 of the substrate 1. Preferably, the metal will be silver or gold, but other metals or alloys could also be used. The deposition of the metal may be performed e.g. by sputtering.

Even if the metal is applied homogeneously over the substrate 1, the surface structures 2 of the substrate 1 will lead to a spatially inhomogeneous deposition of the metal on the substrate surface 4. In particular, the metal gathers preferably on the peaks of the surface structures 2, which leads to the formation of nanoislands or nanoclusters 5 on the peaks of the surface structures 2 as shown in Figure 2. Additionally, metal can gather to form nanoclusters or nanoparticles on the sidewalls of the surface structures, which may also be useful in the optical manipulation of cells. These nanoclusters or nanoparticles 5 may, for example, have dimensions between 100 and 500 nanometers. Their size depends not only on the shape and size of the surface structures 2, but also on the duration and rate of the deposition process. In contrast to conventional methods, no annealing process and no prior generation of individual nanoparticles are required in order to form the nanoclusters or nanoislands 5, thereby making the production of the substrate 1 of the present invention easy and highly reproducible. In particular when using the above described imprint method, the production of the structured and coated substrate 1 is also very cost efficient.

Figure 3 shows the structured and coated substrate 1 of the invention after arranging two biological cells 6 thereon. For example, the cells 6 could be grown on the substrate 1. With its membrane 7, each cell 6 contacts at least one, but preferably several of the metal nanoclusters 5. In order to stabilize the cells 6, and in order to keep them alive, they are surrounded by a suitable medium 8.

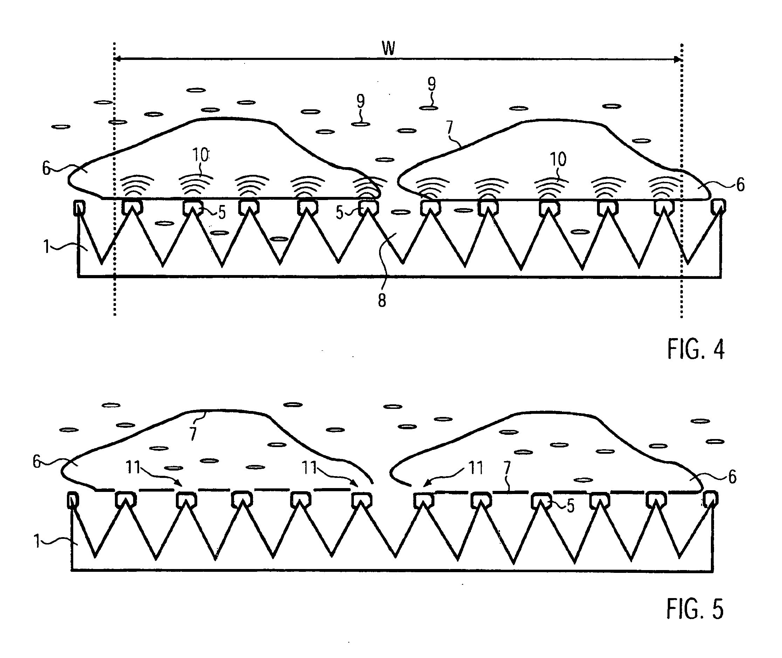

As shown in Figure 4, macromolecules (such as DNA or RNA) or other biologically active or chemical substances 9 (such as medical substances) are distributed in the medium 8 around the cells 6. The cell membrane 7 is strong enough to withstand a penetration by the macromolecules 9.

As also shown in Figure 4, the cells 6 on the substrate 1 are irradiated by at least one laser pulse (typically several laser pulses). The laser pulse may be a femtosecond laser pulse, i.e. with a duration of less than one picosecond, for example with a duration of 50 or 100 femto-seconds. The laser pulse may be delivered e.g. from an Erbium doped fiber laser at a central wavelength of 1,550 nanometers, or a Ytterbium doped fiber laser at a central wavelength of around 1,030 nanometers, or a Ti:Sapphire laser at a central wavelength of 800 nanometers. Other types of femtosecond lasers may also be used. The laser pulses may optionally be amplified and/or (Raman-)shifted or frequency-multiplied. The width W of the laser beam on the substrate 1 is shown schematically in Figure 4. The width W of the laser beam does not have to correspond to a "spot size", as it is not necessary to focus the laser onto the substrate 1. In particular, the width W of the laser beam can be large enough to irradiate several nanoislands or nanoclusters 5 simultaneously, as shown in Figure 4.

During the irradiation of the substrate 1 with the laser pulse, the electromagnetic field of the laser pulse is significantly enhanced at and by the nanoclusters 5. The local enhancement 10 of the electromagnetic field is shown schematically in Figure 4. The field enhancement 10 is strongest at the surface of the nanoclusters 5 themselves, possibly due to surface plasmon generation on the nanoclusters 5 by the laser pulse. The maximum electromagnetic field enhancement relative to the incident laser field preferably can be greater than 100, or greater than 10, or greater than 2.

As shown in Figure 5, the extremely enhanced electromagnetic field 10 at the nanoclusters 5 generates holes 11 in the adjacent cell membrane 7. A hole in the cell membrane can be defined as a change in the permeability of the membrane to external biologically-active or chemical compounds, without necessarily implying a physical opening in the cell membrane. These holes 11 remain open long enough in order to allow the macromolecules 9 to enter into the cells 6 through diffusive or convective processes. Subsequently, the intact cells 6 will close the holes 11 in its cell membrane 7 afterwards.

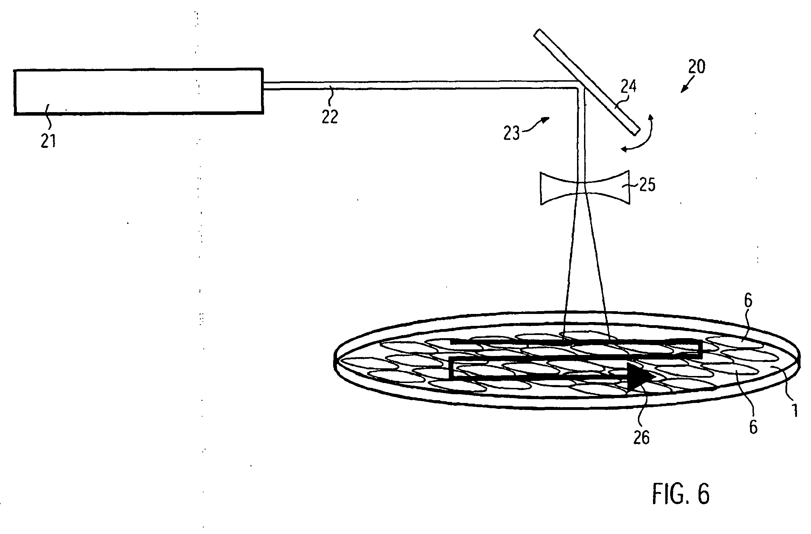

Figure 6 schematically represents a system 20 of the present invention for manipulating biological cells 6. The system 20 comprises a femtosecond laser 21, which delivers a train of ultra-short laser pulses in a beam 22. An optical assembly 23 serves to define the spot size and the position of the laser radiation 22 at the point of interaction with the cells 6. In the embodiment shown in Figure 6, the optical assembly 23 comprises a scanning system with two pivotable scanning mirrors 24 (one of which is shown in Fig. 6) for deflecting the beam 22, as well as a lens (or lens assembly) 25 for defining the spot size of the beam 22. In Figure 6, the lens 25 is a defocusing lens, although the lens could also be a focusing lens.

The structured and coated substrate 1 carrying the biological cells 6 is arranged in a petri dish. As shown in Figure 6, the scanning mirrors 24 may be used to scan the laser beam 22 across the cells 6 in a predetermined scanning pattern 26, such that eventually a predetermined number of femtosecond laser pulses are delivered to each area of the substrate 1, or to each cell 6, respectively. A possible alternative is to use a fixed laser beam and move the sample instead of using a scanning system, or to use a large spotsize/high energy beam, in order to have "single shot flashlight-like exposure".

Starting from the embodiment described above, the method and system of the present invention may be amended in several ways. For example, the average number of nanoislands or nanoclusters 5 per cell 6 can be varied, in order to increase or decrease the manipulation efficiency. As evident from Figure 4, the space between the surface structures 2 significantly enhances the transport of the macromolecules 9 to the area of the cell 6 in which the holes 11 are created, i.e. the side of the cell 6 facing the substrate 1. In order to enhance the transport of macromolecules 9 to these portions of the cell further, the spacing between the surface structures 2 might be increased, or suitable channels (not shown) might be cut into the substrate 1. If desired, the structured and metal coated substrate could be provided with a biocompatible coating, in order to improve cell adhesion.

Further, if the material of the substrate 1 is transparent for the laser radiation, the substrate 1 could also be irradiated from below in order to avoid a distortion of the beam 22 by the medium 8, and by the cells 6. In this way, the transparent substrate 1 might concentrate the laser light towards the peaks of the nanostructures 2, thereby enhancing the electromagnetic field even further. Also, a surface plasmon on the nanostructures 2 could concentrate into a considerably enhanced edge plasmon at the peaks of the nanostructures 2, i.e. at the nanoislands or nanoclusters 5.

In an alternative embodiment, the substrate 1 could be replaced by e.g. a fiber end, allowing in situ transfection inside a human or animal body. Not only the laser can be guided in a pattern, even the surface structuring could already be done in a certain pattern or shape.

In another alternative embodiment, high electric field pulses can be used instead of laser pulses, leading to a modified electroporation method.

Still considered within the present invention, there are several other ways to generate metallic nanostructures over a large area substrate, as alternatives to the specific method described above, for example:
1. metal island films on flat (Si02 or other flat) substrates. These can be fabricated via evaporation (electron beam or thermal) of several (preferably 6, and less than 10) nanometers of gold or silver onto a clean, flat silicon dioxide substrate.
2. Plasmonically-resonant, lithographically (electron beam or photolithography) defined metallic structures; e.g. linear or bow-tie optical antennas, arrays of defined metallic nanoparticles (circles, rods, ellipses, cones, and any combination of such particles, e.g. dimers, trimers, quadramers, etc.) or otherwise), periodically-ruled metal surfaces (such as diffraction grating-type structures);
3. chemically etched surfaces coated with metal, such as "black silicon" surfaces made by reactive-ion etching of silicon, pyramidal structures made by potassium hydroxide etching of silicon, porous silicon, electrochemically-roughened metallic electrodes;
4. colloidal particles chemically or electrostatically bound to the surface of a flat substrate (solid nanoparticles, spherical or otherwise), nanoshells (silica nanospheres coated with a layer of metal), aggregated solid metallic colloidal nanoparticles;
5. metallized polymeric structures fabricated using multi-photon absorption polymerization lithography;
6. metallized polymeric replicas of any of the above - examples of polymeric molding can include soft lithography using PDMS (polydimethylsiloxane), hard-PDMS (also referred to as HPDMS, which allows for smaller structures to be replicated than with standard PDMS soft lithography), nanoimprint lithography, etc.;
7. metal-coated self-assembled surfaces, fabricated using a nanosphere lithography, used for fabricating hexagonal close-packed monolayers of polystyrene (or other non-metallic material) spheres on a flat substrate. These spheres can be coated with a film of metal via evaporation or sputtering, and used for the cell manipulation as coated. Alternatively, the spheres can be used as a mask to be removed, leaving behind metallic nanoparticles in the interstices of the spheres on the surface of the substrate. These remaining particles can themselves be used to enhance the local electromagnetic field for use in cell manipulation.

## Claims

1. Method for the manipulation of at least one cell (6), the method comprising the following steps:
- depositing a metal onto the surface (4) of a substrate (1),
- placing the at least one cell (6) at or near the surface (4) of the substrate (1),
- irradiating the surface (4) of the substrate (1) with at least one laser pulse,
**characterized in that**
surface structures (2) with a size of 1 micrometer or less are formed on the surface (4) of the substrate (1) prior to depositing the metal thereon,
**in that** nanoislands or nanoclusters (5) are formed on peaks of the surface structures (2) by depositing the metal thereon,
and **in that** the at least one laser pulse is a picosecond pulse or a femtosecond pulse.

2. Method according to claim 1, wherein the surface structures (2) are formed by laser ablation from the surface (4) of the substrate (1), or by taking an imprint from a previously formed die.

3. Method according to any of the preceding claims, wherein the substrate (1) comprises a plastic material or a semiconductor material.

4. Method according to any of the preceding claims, wherein a plurality of cells (6) is immobilized on the substrate (1).

5. Method according to any of the preceding claims, wherein a plurality of surface structures (2) are irradiated by each laser pulse.

6. Method according to any of the preceding claims, wherein consecutive laser pulses are guided across the surface (4) of the substrate (1) in a predetermined or random pattern (26).

7. System (20) for the manipulation of at least one cell (6), the system (20) comprising a substrate (1) with surface structures (2) having a size of 1 micrometer or less, wherein a metal is deposited onto the surface structures (2) so as to form nanoislands or nanoclusters (5) on peaks of the surface structures (2),
wherein the system (20) further comprises a laser (21) for irradiating the surface structures (2) of the substrate (1),
and wherein the laser (21) is a picosecond laser or a femtosecond laser.

8. System according to claim 7, wherein the substrate (1) comprises a plastic material or a semiconductor material.

9. System according to one of claims 7 or 8, wherein the substrate material is transparent for the laser radiation.

10. System according to one of claims 7 to 9, wherein more than 75% of the surface structures (2) on a given area of the substrate have a size of less than 700 nm, preferably less than 500 nm.

11. System according to one of claims 7 to 10, wherein the metal deposited on the substrate (1) is gold, silver, copper or aluminium.

12. System according to one of claims 7 to 11, further comprising an optical assembly (23) for defining the spot size and the position of the laser radiation on the substrate surface (4).

13. System according to one of claims 7 to 12, wherein channels in the substrate (1) are provided for enhancing the transport of macromolecules (9) to the cell (6).

14. System according to one of claims 7 to 13, wherein a biocompatible coating is provided on the structured and metal coated substrate (1), in order to improve cell adhesion.

15. Use of a method according to any of claims 1 to 6 or of a system (20) according to any of claims 7 to 14 for the manipulation of at least one cell (6), in particular for transfection of at least one cell (6).

## Patentansprüche

1. Verfahren zur Manipulation wenigstens einer Zelle (6), wobei das Verfahren folgende Schritte aufweist:
- Ablagern eines Metalls auf der Oberfläche (4) eines Substrats (1),
- Platzieren der wenigstens einen Zelle (6) auf oder nahe der Oberfläche (4) des Substrats (1),
- Bestrahlen der Oberfläche (4) des Substrats (1) mit wenigstens einem Laserpuls,
**dadurch gekennzeichnet,**
**dass** auf der Oberfläche (4) des Substrats (1) Oberflächenstrukturen (2) mit einer Größe von einem Mikrometer oder weniger gebildet sind, bevor das Metall darauf abgelagert wird,
**dass** auf den Spitzen der Oberflächen (2) durch das Ablagern des Metalls darauf Nanoinseln oder Nanocluster (5) gebildet werden,
und **dass** der wenigstens eine Laserpuls ein Pikosekunden-Puls oder ein Femtosekunden-Puls ist.

2. Verfahren nach Anspruch 1, wobei die Oberflächenstrukturen (2) gebildet werden durch Laserabtrag von der Oberfläche (4) des Substrats (1), oder durch das Abnehmen eines Abdrucks von einem vorgeformten Werkzeug.

3. Verfahren nach einem der vorangehenden Ansprüche, wobei das Substrat (1) ein Kunststoffmaterial oder ein Halbleitermaterial aufweist.

4. Verfahren nach einem der vorangehenden Ansprüche, wobei eine Vielzahl von Zellen (6) auf dem Substrat (1) imobilisiert wird.

5. Verfahren nach einem der vorangehenden Ansprüche, wobei eine Vielzahl von Oberflächenstrukturen (2) von jedem Laserpuls bestrahlt werden.

6. Verfahren nach einem der vorangehenden Ansprüche, wobei aufeinanderfolgende Laserpulse in einem vorgegebenen Muster oder in einem Zufallsmuster (26) über die Oberfläche (4) des Substrats (1) geführt werden.

7. System (20) zur Manipulation wenigstens einer Zelle (6), wobei das System (20) wenigstens ein Substrat (1) mit Oberflächenstrukturen (2) mit einer Größe von einem Mikrometer oder weniger aufweist, wobei ein Metall abgelagert ist auf den Oberflächenstrukturen (2), sodass auf den Spitzen der Oberflächenstrukturen (2) Nanoinseln oder Nanocluster (5) gebildet sind,
wobei das System (20) ferner einen Laser (21) aufweist zum Bestrahlen der Oberflächenstrukturen (2) des Substrats (1),
und wobei der Laser (21) ein Pikosekunden-Laser oder Femtosekunden-Laser ist.

8. System nach Anspruch 7, wobei das Substrat (1) ein Kunststoffmaterial oder ein Halbleitermaterial aufweist.

9. System nach einem der Ansprüche 7 oder 8, wobei das Substratmaterial für die Laserstrahlung transparent ist.

10. System nach einem der Ansprüche 7 bis 9, wobei mehr als 75% der Oberflächenstrukturen (2) auf einem vorgegebenen Gebiet des Substrats eine Größe von weniger als 700 nm, vorzugsweise von weniger als 500 nm aufweisen.

11. System nach einem der Ansprüche 7 bis 10, wobei das auf dem Substrat (1) abgelagerte Metall Gold, Silber, Kupfer oder Aluminium ist.

12. System nach einem der Ansprüche 7 bis 11, ferner umfassend eine Optikanordnung (23) zum Definieren einer Spotgröße und der Position der Laserstrahlung auf der Substrat-Oberfläche (4).

13. System nach einem der Ansprüche 7 bis 12, wobei im Substrat (1) Kanäle vorgesehen sind zum Unterstützen des Transports von Makromolekülen (9) zu der Zelle (6).

14. System nach einem der Ansprüche 7 bis 13, wobei eine biokompatible Beschichtung auf dem strukturierten und metallbeschichteten Substrat (1) vorgesehen ist, um die Adhäsion von Zellen zu verbessern.

15. Verwendung eines Verfahrens nach einem der Ansprüche 1 bis 6 oder eines Systems (20) nach einem der Ansprüche 7 bis 14 für die Manipulation wenigstens einer Zelle (6), insbesondere für die Transfektion wenigstens einer Zelle (6).

## Revendications

1. Procédé de manipulation d'au moins une cellule (6), le procédé comprenant les étapes suivantes :
dépôt d'un métal sur la surface (4) d'un substrat (1),
placement de l'au moins une cellule (6) sur ou près de la surface (4) du substrat (1),
irradiation de la surface (4) du substrat (1) avec au moins une impulsion laser,
**caractérisé en ce que**
des structures de surface (2) d'une taille inférieure ou égale à 1 µm sont formées sur la surface (4) du substrat (1) avant dépôt du métal sur celle-ci,
**en ce que** des nanoîlots de nanogroupements (5) sont formés sur les crêtes des structures de surface (2) par dépôt du métal sur celles-ci,
et **en ce que** l'au moins une impulsion laser est une impulsion picoseconde ou une impulsion femtoseconde.

2. Procédé selon la revendication 1, dans lequel les structures de surface (2) sont formées par ablation par laser de la surface (4) du substrat (1), ou en prenant une empreinte d'une matrice précédemment formée.

3. Procédé selon l'une quelconque des revendications précédentes, dans lequel le substrat (1) comprend une matière plastique ou une matière semiconductrice.

4. Procédé selon l'une quelconque des revendications précédentes, dans lequel une pluralité de cellules (6) sont immobilisées sur le substrat (1).

5. Procédé selon l'une quelconque des revendications précédentes, dans lequel une pluralité de structures de surface (2) sont irradiées par chaque impulsion laser.

6. Procédé selon l'une quelconque des revendications précédentes, dans lequel des impulsions laser consécutives sont guidées sur la surface (4) du substrat (1) selon un motif prédéterminé ou aléatoire (26).

7. Système (20) de manipulation d'au moins une cellule (6), le système (20) comprenant un substrat (1) avec des structures de surface (2) d'une taille inférieure ou égale à 1 µm, dans lequel un métal est déposé sur les structures de surface (2) afin de former des nanoîlots de nanogroupements (5) sur les crêtes des structures de surface (2),
dans lequel le système (20) comprend en outre un laser (21) pour irradier les structures de surface (2) du substrat (1),
et dans lequel le laser (21) est un laser picoseconde ou un laser femtoseconde.

8. Système selon la revendication 7, dans lequel le substrat (1) comprend une matière plastique ou une matière semiconductrice.

9. Système selon l'une des revendications 7 ou 8, dans lequel le matériau du substrat est transparent au rayonnement laser.

10. Système selon l'une des revendications 7 à 9, dans lequel plus de 75 % des structures de surface (2) sur une aire donnée du substrat ont une taille inférieure à 700 nm, de préférence inférieure à 500 nm.

11. Système selon l'une des revendications 7 à 10, dans lequel le métal déposé sur le substrat (1) est de l'or, de l'argent, du cuivre ou de l'aluminium.

12. Système selon l'une des revendications 7 à 11, comprenant en outre un montage optique (23) pour définir la taille de spot et la position du rayonnement laser sur la surface du substrat (4).

13. Système selon l'une des revendications 7 à 12, dans lequel des canaux sont prévus dans le substrat (1) pour améliorer le transport de macromolécules (9) vers la cellule (6).

14. Système selon l'une quelconque des revendications 7 à 13, dans lequel un revêtement biocompatible est prévu sur le substrat structuré et recouvert de métal (1), pour améliorer l'adhérence des cellules.

15. Utilisation d'un procédé selon l'une quelconque des revendications 1 à 6 ou d'un système (20) selon l'une quelconque des revendications 7 à 14 pour la manipulation d'au moins une cellule (6), en particulier pour la transfection d'au moins une cellule (6).
